(19) Europäisches Patentamt
European Patent Office
Office européen des brevets

(11) Numéro de publication: **0 061 953**
**A1**

(12) **DEMANDE DE BREVET EUROPEEN**

(21) Numéro de dépôt: 82400464.2

(22) Date de dépôt: 12.03.82

(51) Int. Cl.³: C 12 P 21/00
//C12R1/91

(30) Priorité: 23.03.81 FR 8105764

(43) Date de publication de la demande: 06.10.82 Bulletin 82/40

(84) Etats contractants désignés: BE CH DE GB LI NL

(71) Demandeur: CENTRE NATIONAL DE LA RECHERCHE SCIENTIFIQUE
15, Quai Anatole France
F-75007 Paris(FR)

(71) Demandeur: INSTITUT PASTEUR
25-28, rue du Docteur Roux
F-75724 Paris Cedex 15(FR)

(72) Inventeur: Montagnier, Luc
21, rue de Malabry
F-92350 Le Plessis Robinson(FR)

(72) Inventeur: Svab, Josette
8, Avenue Félix Faure
F-75015 Paris(FR)

(72) Inventeur: Meurs-Sylvestre, Eliane
192, rue Lecourbe
F-75015 Paris(FR)

(74) Mandataire: Gutmann, Ernest et al,
Cabinet Plasseraud 84, rue d'Amsterdam
F-75009 Paris(FR)

(54) **Perfectionnements aux procédés de fabrication d'interféron.**

(57) L'invention concerne un procédé perfectionné de production d'interféron. Il consiste à produire l'incubation d'une culture de cellules productrices d'interféron en présence d'un dérivé de la vitamine A, tel que l'acide rétinoïque, préalablement à l'introduction dans la culture d'un inducteur de la synthèse d'interféron par les cellules de la culture.

EP 0 061 953 A1

PERFECTIONNEMENTS AUX PROCEDES DE FABRICATION D'INTERFERON

L'interféron est formé d'au moins une protéine dont la synthèse par les cellules est généralement induite par une infection virale. Il est bien connu que l'interféron est capable d'inhiber le développement des virus, qu'il s'agisse de ceux ayant provoqué l'induction de la synthèse ou d'autres virus, même si l'efficacité d'inhibition de l'interféron ainsi synthétisé peut être variable d'un type de virus à un autre. L'interféron synthétisé par les cellules est normalement excrété dans le milieu extracellulaire. Il peut être absorbé par d'autres cellules et leur conférer ainsi une protection à l'égard d'une infection virale. L'interféron peut aussi, en particulier lorsque les cellules productrices sont cultivées *in vitro* au sein d'un milieu, de préférence liquide, être en principe récupéré à partir de celui-ci.

On sait en effet que l'interféron représente un principe actif de grande valeur en thérapeutique, en raison de ses propriétés antivirales. Il peut être utilisé avec succès, par exemple pour le traitement d'infections dues au virus de l'herpès. On fonde en outre de grands espoirs sur la capacité attendue de l'interféron d'inhiber le développement de certains types de cancer.

L'invention concerne donc plus particulièrement des perfectionnements apportés aux procédés de production d'interféron, ou plus généralement d'interférons car, comme cela est bien connu également, il existe plusieurs formes d'interféron selon le type des cellules productrices mises en oeuvre. Parmi les cellules productrices les plus couramment mises en oeuvre, on peut citer les leucocytes du sang, les lymphocytes, les fibroblastes, les cellules lymphoblastoïdes, des cellules épithéliales, par exemple des cellules de rein, ou encore des cellules HeLa, des cellules de myélome, etc.

L'invention s'intéresse plus particulièrement à la production d'interférons utilisables chez l'homme, par

conséquent à des interférons produits par des cellules d'origine humaine.

On connaît les difficultés sérieuses qui limitent l'efficacité des procédés connus de fabrication d'interféron. Même si l'on fait abstraction des difficultés bien connues que rencontre la culture *in vitro* de cellules d'origine humaine, il en est une qui présente une caractéristique prédominante : il s'agit des quantités extrêmement faibles d'interféron qui sont excrétées dans les milieux de culture, mis en oeuvre par la plupart des procédés connus de production d'interféron.

L'invention a donc pour but de remédier au moins en partie aux difficultés rencontrées à l'occasion de la mise en oeuvre des procédés connus, notamment du type de ceux dans lesquels on a recours à un agent inducteur introduit dans une culture de cellules capables de produire de l'interféron, dès lors qu'elles sont susceptibles de le synthétiser sous l'effet d'un tel inducteur.

Le procédé selon l'invention est caractérisé en ce que lesdites cellules sont mises en contact avec de l'acide rétinoïque, du type de la vitamine A ou de l'un de ses analogues ou dérivés (ces différents composés étant ci-après désignés dans leur ensemble sous l'expression "dérivés rétinoïques"), puis débarrassées de ces dérivés rétinoïques, préalablement à la mise en contact desdites cellules avec l'inducteur.

De préférence, le dérivé rétinoïque mis en oeuvre est constitué par l'acide rétinoïque (acide diméthyl-3,5 (triméthyl-2,6,6-cyclohexane-1 yl 1)-9 nonatétraène-2,4,6,8-oïque) de formule :

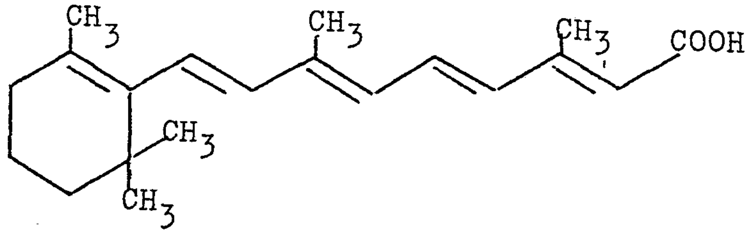

A titre d'exemples d'autres dérivés rétinoïques susceptibles d'être mis en oeuvre dans le procédé conforme à l'invention, on citera les homologues de cet acide rétinoïque, cités à la page 35 de l'article publié par Reuben Lotan dans "Biochimica et Biophysica Acta", 605 (1980) 33-91, sous le titre "Effects of vitamin A and its analogs (retinoids) on normal and neoplastic cells" (effets de la vitamine A et de ses analogues (rétinoïdes) sur des cellules normales et néoplasiques).

Comme le montre le tableau figurant dans cet article, le groupe terminal cyclique et la chaîne polyénique de l'acide rétinoïque peuvent faire l'objet de nombreuses modifications ou substitutions, le groupe carboxylique pouvant lui-même être remplacé par d'autres groupes, notamment alcools, aldéhydes, hétéroxydes, méthyl, alcoyles inférieurs, etc. De préférence, on a recours à ceux des dérivés ou analogues en cause, dans lesquels la chaîne polyénique est terminée par un groupe carboxylique, à l'instar de l'acide rétinoïque lui-même, dont la formule a été indiquée ci-dessus.

En ce qui concerne les cellules productrices d'interféron, on pourra avoir recours à toutes lignées de cellules, de préférence d'origine humaine, susceptibles de produire de l'interféron. Ces cellules appartiennent avantageusement à l'une des lignées cellulaires déjà identifiées plus haut. Les lignées de cellules Namalva, bien connues des spécialistes, telles que celles qui sont dérivées de cellules de patients affectés par le lymphome de Burkitt sont à ce jour préférées. A titre d'exemple de cellules Namalva susceptibles d'être utilisées, on citera celles dénommées Namalva/WRL qui sont disponibles à l'American Type Culture Collection (A.T.C.C. CRL 1432).

En ce qui concerne l'inducteur, on peut avoir recours également à tout type d'inducteur classiquement utilisé, à savoir des virus tels que le virus Sendaï ou le virus NDV (Newcastle Disease Virus) qui peuvent éventuellement être inactivés, ou encore des polynucléo-

tides ou polyribonucléotides, par exemple l'acide poly-inosinique : polycytidylique (poly I - poly C).

La découverte que la mise en contact des cellules productrices d'interféron avec un dérivé de la famille rétinoïque ou leur incubation en présence de ce dérivé, préalablement à leur mise en contact avec un inducteur, pouvait conduire à un net accroissement de la quantité d'interféron susceptible d'être produite et excrétée par lesdites cellules dans un milieu de culture approprié, était d'autant plus inattendue qu'était connue la propriété que possède la vitamine A d'inhiber la production d'interféron par des cellules productrices, lorsqu'elle est mise en contact avec celles-ci, préalablement ou simultanément à la mise en contact avec un inducteur du type de ceux déjà mentionnés, comme cela a été décrit par plusieurs auteurs, notamment par J.E. Blalock et G.E. Gifford dans un article intitulé "Inhibition of interferon action by vitamin A" (inhibition de l'action de l'interféron par la vitamine A), paru dans "J. Gen. Virol." (1975), 29, 315-324 ou dans l'article de J.E. Blalock et G.E. Gifford intitulé "Suppression of interferon production by vitamin A" (Suppression de la production de l'interféron par la vitamine A), paru dans "J. Gen. Virol." (1976), 32, 143-147.

Cette capacité de la vitamine A d'inhiber la production de l'interféron de cellules, préalablement ou simultanément mises en contact avec un inducteur, est d'ailleurs considérée comme une connaissance acquise de l'état de la technique, comme en témoigne la publication extrêmement complète déjà identifiée plus haut de Reuben Lotan.

Avantageusement, la mise en contact est réalisée avec le milieu de culture des cellules productrices d'interféron, lorsque le niveau de développement de la culture a atteint le stade désiré. En effet, on aura recours à une concentration du dérivé de l'acide rétinoïque aussi élevée que possible, compatible avec la viabilité de la structure cellu-

laire, de préférence néanmoins proche de la concentration cytostatique. A cet égard, on remarquera que l'on peut naturellement avoir recours à des concentrations plus faibles de dérivés rétinoïques. La concentration finale de l'interféron produit dans le milieu en sera cependant amoindrie. Lorsque les cellules mises en oeuvre appartiennent à la lignée Namalva et lorsque le dérivé rétinoïque utilisé est constitué par l'acide rétinoïque, les concentrations de ce dernier seront avantageusement ajustées de $1.10^{-6}$ à $1.10^{-4}$M. Il ne s'agit cependant là que d'un intervalle indiqué à titre d'exemple. Il appartient en fait à l'homme de l'art de déterminer les doses les plus efficaces, notamment en ayant recours à une série d'essais avec des doses croissantes permettant d'explorer celles qui permettront, compte tenu du type de dérivé rétinoïque utilisé et du type de cellules productrices d'interféron mises en oeuvre, d'établir la zone de concentration la plus efficace.

En particulier, on recherchera les doses relatives de l'agent rétinoïque utilisé, susceptibles de conduire à une quasi-interruption de la croissance cellulaire, sans pour autant porter atteinte à la viabilité d'une proportion majeure des cellules alors présentes dans la culture.

De même, les durées de contact entre le dérivé rétinoïque et la culture peuvent être déterminées par l'expérience. De préférence, ces durées de contact sont d'au moins 24 heures, de préférence de l'ordre de 48 heures.

Les cellules ainsi traitées, préalablement débarrassées du dérivé rétinoïque, notamment par lavage ou tout autre mode de séparation, peuvent alors être mises en contact avec l'inducteur.

On peut avoir recours pour la mise en oeuvre du procédé selon l'invention, à tout milieu de culture classique considéré comme approprié pour le développement des cellules productrices d'interféron.

D'autres caractéristiques de l'invention apparaîtront encore au cours d'un exemple de mise en oeuvre préférée de l'invention, qui n'est cependant donné ci-après qu'à titre d'illustration complémentaire non limitative du procédé perfectionné faisant l'objet de cette invention.

1°) Conditions de culture :

Les cellules Namalva sont cultivées en suspension non agitée (sauf si autrement indiqué). dans le milieu de culture RPMI 16.40 additionné de 10 % de sérum de veau foetal (RETAHUIN, ARMOUR) et d'antibiotiques (CHLORTETRACYCLINE 5 µg/ml, AMPHOTERICIN B : 1,5 µg/ml) dans des flacons inclinés de façon à permettre l'oxygénation du milieu. Le volume total du milieu ainsi complété est de 400 ml.

2°) Prétraitement à l'acide rétinoïque

48 heures avant l'induction, on laisse sédimenter les cellules par gravité durant 6 h à 37°C, le flacon étant redressé verticalement.

On enlève ensuite les 3/4 du milieu, soit 300 ml, on compte les cellules et on ajoute du milieu frais pour avoir une concentration de $10^6$ cellules /ml.

On ajoute $5.10^{-5}$M d'acide rétinoïque (SIGMA) à partir d'une solution mère dans l'éthanol à $10^{-2}$M, pour obtenir une concentration finale de $5.10^{-5}$M , soit 0,015 mg/ml. Les flacons sont remis en position inclinée et incubés à 37°C pendant 48 heures à l'abri de la lumière.

On agite manuellement et périodiquement quelques secondes, pour remettre en suspension les cellules.

3°) Induction :

Après 48 heures, on centrifuge les cellules pendant 5 minutes à 1000 tours/minute à 4°C. Le surnageant est éliminé. Le culot cellulaire est resuspendu en milieu RPMI sans sérum, mais contenant, outre les antibiotiques, 0,5 % (concentration finale) de sérumalbumine humaine et $10^{-5}$M de β-mercapto-éthanol, de façon que la concentration cellulaire soit de $3,3.10^6$/ml, soit une réduction de trois fois du volume de

milieu initial : les cellules sont restées en effet à la concentration de $10^6$/ml, leur multiplication ayant été bloquée par l'acide rétinoïque.

On ajoute ensuite 500 unités hémagglutinantes (unités HA) de virus de Sendaï (préalablement inactivé par irradiation UV avec une lampe germicide à la dose de 300 ergs/$mm^2$) par ml de suspension cellulaire. On incube pendant environ 17 heures à 37°C en flacons Corning de 75 $cm^2$, à raison de 100 ml par flacon.

4°) Récolte :

On centrifuge la suspension 10 minutes à 2000 tours par minute à 4°C. On amène le surnageant à pH 2 par addition de HCl 1N et on laisse pendant trois à quatre jours. On ramène ensuite la solution à pH 7 par addition de NaOH 4N. Un aliquot est prélevé pour titrage : les titres vont de 0,5 à $1,2.10^6$ unités internationales/ml (les unités internationales sont définies d'après un interféron leucocytaire de référence n° 6.023.901-527 du NIH). (A titre de comparaison, on indique qu'une préparation d'interféron réalisée dans des conditions identiques, sauf que les cellules mises en oeuvre n'ont pas, avant induction, été pré-incubées en présence d'acide rétinoïque, conduit, au même stade de la préparation, à une solution titrant de $1,5.10^4$ à $6.10^4$ unités internationales/ml).

Le reste du surnageant reçoit de l'acide trichloracétique (concentration finale 1,5 %, à partir d'une solution à 50 %), qui précipite la majorité des protéines, dont l'interféron. Après 60 minutes à 4°C, on centrifuge le précipité à 10.000 tours/minute pendant 15 minutes. Le culot est redissous dans un volume minimal (environ 1/16 du volume initial de tampon phosphate PBS (Proc. Nat. Acad. Sci. 1952, 38, 747), en remontant progressivement le pH à 7 par addition de NaOH 4 N. Le PBS utilisé selon l'invention ne contient en outre, ni calcium, ni magnésium. Un aliquot est prélevé pour titrage.

On peut éventuellement dialyser contre du PBS ou un autre tampon en vue d'une étape ultérieure de purification selon une technique en soi connue.

Des expériences menées dans les mêmes conditions avec du palmitate de rétinol (concentration optimale : $5.10^{-4}$M) et du rétinal (concentration optimale : $5.10^{-5}$M) ont donné des résultats identiques à ceux obtenus avec l'acide rétinoïque.

REVENDICATIONS

1 - Procédé de fabrication d'interféron par culture de cellules productrices d'interféron dans un milieu de culture de préférence liquide, et mise en contact de la culture avec un inducteur d'interféron, caractérisé en ce que les cellules de cette culture sont mises en contact avec un dérivé rétinoïque, puis débarassées de ce dérivé rétinoïque, préalablement à leur mise en contact ultérieure avec l'inducteur.

2 - Procédé selon la revendication 1, caractérisé en ce que le dérivé rétinoïque est un dérivé acide.

3 - Procédé selon la revendication 2, caractérisé en ce que le dérivé rétinoïque acide est constitué par l'acide rétinoïque.

4 - Procédé selon l'une quelconque des revendications 1 à 3, caractérisé en ce que les concentrations relatives du milieu de culture en dérivé rétinoïque sont ajustées aux doses permettant une quasi-interruption de la croissance cellulaire, sans pour autant porter atteinte à la viabilité d'une proportion majeure des cellules alors présentes dans la culture.

5 - Procédé selon la revendication 4, caractérisé en ce que la durée de contact entre la culture et le dérivé rétinoïque est d'au moins 24 heures, de préférence de l'ordre de 48 heures.

6 - Procédé selon les revendications 3, 4, 5 et 6 prises dans leur ensemble, caractérisé en ce que la concentration en acide rétinoïque du milieu de culture est réglée à une valeur de $1.10^{-6}$ à $1.10^{-4}$M.

7. Procédé selon l'une quelconque des revendications 1 à 6, caractérisé en ce que les cellules productrices d'interféron mises en oeuvre appartiennent à une lignée de Namalva.

8 - L'interféron préparé selon le procédé défini dans l'une quelconque des revendications 1 à 7 .

0061953

Office européen des brevets

# RAPPORT DE RECHERCHE EUROPEENNE

Numéro de la demande

EP 82 40 0464

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D-X | CHEMICAL ABSTRACTS, vol. 85, no. 13, 27 septembre 1976, page 29, référence 87190p COLUMBUS OHIO (US) & J. Gen. Virol. 1976, 32 Pt. 1, 143-7. J.E. BLALOCK et al.: "Suppression of interferon production by vitamin A". * en entier * | 1-8 | C 12 P 21/00// C 12 R 1/91 |
| A | EP - A - 0 000 520 (THE WELLCOME FOUNDATION) * revendications * | 1-8 | |

| DOMAINES TECHNIQUES RECHERCHES (Int. Cl. 3) |
|---|
| C 12 P |
| C 12 R |
| A 61 K |

Le présent rapport de recherche a été établi pour toutes les revendications

| Lieu de la recherche | Date d'achèvement de la recherche | Examinateur |
|---|---|---|
| La Haye | 22-06-1982 | RIJCKEBOSCH |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503. 03 82